# EUROPEAN PATENT APPLICATION

(11) **EP 1 331 481 A1**
(43) Date of publication of application: **30.07.2003**
(21) Application number: 01978949.4
(22) Date of filing: 30.10.2001
(51) Int. Cl.: G01N 33/53

(54) **DIAGNOSTIC KIT FOR SCHIZOPHRENIA**

(30) Priority: 31.10.2000 JP 2000331742
(71) Applicant: Japan as represented by President of Niigata University, Niigata-shi, Niigata 950-2181 (JP)
(72) Inventor: NAWA, Hiroyuki, Niigata-shi, Niigata 951-8104 (JP); FUTAMURA, Takashi, Niigata-shi, Niigata 950-2002 (JP); SOMEYA, Toshiyuki, Niigata-shi, Niigata 950-2045 (JP); ASAMA, Koue, Sanjo-shi, Niigata 955-0082 (JP)
(74) Representative: Wibbelmann, Jobst, Dr., Dipl.-Chem.
(86) International application number: JP0109514
(87) International publication number: WO02037105

(57) **Abstract**

According to the present invention, a diagnostic kit for schizophrenia was provided and the diagnostic kit comprises measurement of serum epidermal growth factor content using anti-epidermal growth factor antibody. The diagnosis kit according to this invention is useful for objective diagnosis of schizophrenia.

## Description

### BACKGROUND OF THE INVENTION

### 1. Field of the invention

This invention relates to a diagnostic kit for schizophrenia. More specifically, this invention relates to a diagnostic kit for schizophrenia comprising an antibody against epidermal growth factor (EGF).

### 2. Prior art

Schizophrenia appears from adolescence to manhood with characteristic symptoms in perception, cerebration, emotion and behavior. In many cases, the progression of this disease is a chronic process accompanied with various difficulties in social adaptation. With regard to schizophrenia, there are classifications of positive symptoms (hallucination, delusion, diminished cerebration, tension and curious behavior, etc.) and negative symptoms (flattening in emotion, decrease in will and social withdrawal, etc.). As stated above, the present diagnostics of schizophrenia is defined only by psychological symptoms of a patient. Then it is markedly affected by subjective view of a doctor who conducts the diagnosis. Therefore, problems on the objectivity of the diagnosis have been recited many times. Socially, because of the specific pathology of this disease, establishment of a consistent and comprehensive treatment system against this disease, such as early detection of occurrence, treatment and social reversion at early stage, and prevention of recurrence, has been desired. For treatment of schizophrenia, medical therapy (in many cases, long term administration for years) is indispensable. Then, phenothiazine, thioxanthene derivatives, butyrophenone derivatives, benzamide derivatives, and serotonine dopamine antagonists have been administered to patients.

If there are some biochemical alterations, which are regulated by genetics, involved in schizophrenia, how it can be proved? In the case of the abnormality in an enzyme existing in the whole body, such as congenital abnormality in amino acid metabolism or carbohydrate metabolism, identification of such enzyme can be performed easily using blood or urine. From the level of the amino acid or the level of its metabolite in blood, the enzyme responsible for the abnormality can be identified. Moreover, using blood cells or cultured tissues of skin or using materials from autopsy, this abnormality in the enzyme can be proved in many cases. Plenty of researches have been performed investigations on body fluids of schizophrenic patients, but any apparent abnormality has not yet been found until now. There were some reports describing that a certain substance was specifically found out in blood or urine of schizophrenic patent. However, almost all of them were denied when trace experiments were repeated.

On the other hand, epidermal growth factor (EGF) has been researched as a malignant factor, which is involved in growth of cancers or benign tumors. However, there has been no knowledge on the role of EGF in schizophrenia, which is a psychotic disease. The present invention has firstly elucidated the relationship between EGF concentration in serum and schizophrenia.

### SUMMARY OF THE INVENTION

Schizophrenia appears 0.7-1.0% persons of population, and it is a serious disease which occupies about 40% of mental disorders. Irrespective of the above, biochemical diagnostic method of schizophrenia has not been established. Thus, development of a diagnostic kit, available for detection of biochemical alteration due to schizophrenia, has been desired in the medical field. Therefore, the object of the present invention is to provide such a diagnostic kit for schizophrenia.

The present inventors have earnestly investigated to solve the above-mentioned object. As a result, the inventors found that, the level of serum EGF significantly decreased in chronic schizophrenic patents and acute schizophrenic patients as compared with the normal person, and the brain content of EGF significantly decreased in chronic schizophrenic patients by the examination using postmortem brain samples, whereby the inventors have accomplished the present invention.

In the following, this invention is explained in detail, however, these detailed explanation and the examples do not intend to restrict or limit the effective range of this invention.

### BRIEF DESCRIPTION OF THE DRAWINGS

Fig. 1 is a graph showing EGF content in blood serum, in comparison of the control volunteer group of volunteers and the chronic schizophrenic group of human beings.
Fig. 2 is a graph showing EGF content in blood serum, in comparison of the control group and the haloperidol administered group of rats.
Fig. 3 is a graph showing distribution of EGF content in blood serum indicated by logarithmic value, in comparison of the control volunteer group and the chronic schizophrenic group of human beings.
Fig. 4 is a graph showing EGF content in blood serum, in comparison of the control volunteer group and the drug-free chronic schizophrenic group of human beings.

### DETAILED EXPLANATION OF THE PREFERRED EMBODIMENTS

The present invention relates to a diagnostic kit for schizophrenia, wherein serum is prepared from human blood and the EGF content is measured by various methods. In the method of the present invention, EGF is preferably detected by sandwich ELISA (Enzyme-linked immunosorbent assay), a method highly specific to EGF. In serum of schizophrenic patients, EGF concentration significantly decreased as compared with normal person, and the method utilizes this knowledge.

More specifically, the present invention is a diagnostic kit for schizophrenia, comprising a solid phase, an anti-epidermal growth factor (EGF) antibody immobilized to said solid phase and a labeled anti-EGF antibody. In the present invention, the anti-EGF antibody is labeled by enzyme labeling, fluorescent labeling or radioisotope labeling, etc. Here, the enzyme used for said enzyme labeling are peroxidase, β-D-galactosidase, alkaline phosphatase and glucose-6-phosphate dehydrogenase. The diagnostic kit of the present invention may also provide a detecting reagent for detection of the labeling of said labeled anti-EGF antibody, if necessary.

Moreover, the present invention is a diagnostic kit for schizophrenia comprising a solid phase, an anti-EGF andibody immobilized to said solid phase, a biotin-modified anti-EGF antibody and labeled avidine which reacts with said biotin. Moreover, the present invention is a diagnostic kit for schizophrenia comprising a solid phase, an anti-EGF antibody immobilized to the solid phase, a 2,4-dinitrophenol modified anti-EGF antibody and a labeled anti-2,4-dinitrophenol antibody which reacts with said 2,4-dinitrophenol. The diagnostic agent kit of the present invention may also provides a detecting reagent for detection of the labeling of said labeled avidin or said labeled 2,4-dinitrophenol antibody, if necessary. As mentioned above, the diagnostic kit of this invention is characterized in that EGF concentration in serum of the patient is measured using said anti-EGF antibody. The assay kit for schizophrenia of the present invention is effective for diagnosis of acute schizophrenia or chronic schizophrenia.

In the following, the meaning or definition of the term recited in the present specification is described.

The "anti-epidermal growth factor antibody" means an antibody prepared using epidermal growth factor (abbreviated to as "EGF") as the antigen. Said antibody may be any antibody, so long as it is capable of binding to EGF, and both of polyclonal antibodies and monoclonal antibodies can be included. Moreover, polyclonal antibodies and monoclonal antibodies capable of specifically binding to EGF are preferred.

The "labeled anti-EGF antibody" means an antibody in which the anti-EGF antibody is labeled with an enzyme such as peroxidase, β-D-galactosidase, alkaline phosphatase and glucose-6-phosphate dehydrogenase, otherwise with a fluorescent such as delfinium or a radioisotope. As a result of the labeling, the amount of the anti-EGF antibody can be quantified. When labeled by an enzyme, the bound anti-EGF antibody can be detected by reacting said enzyme with a suitable substrate, using the enzyme reaction product as a marker. Moreover, in the case of fluorescent labeling or radioisotope labeling, the bound anti-EGF antibody can be detected, using the fluorescence or the radioactivity as a marker.

Moreover, in the "labeled anti-EGF antibody", anti-EGF antibodies labeled with biotin, 2,4-dinitrophenol and etc. are also included. Biotin specifically binds to avidin, and 2,4-dinitrophenol specifically binds to anti-2,4-dinitrophenol antibody. Thus, the above-mentioned labeled anti-EGF antibody can be quantified by avidin labeled with an enzyme such as peroxidase, β-D-galactosidase, alkaline phosphatase and glucose-6-phosphate dehydrogenase and etc., or by anti-2,4-dinitrophenol antibody.

The "schizophrenia" means "endogenic psychological disiase mainly appears at adolescence, which is a serious disease in that many cases go through chronic pathology and one's personality is gradually disrupted and part of the patients are forced to reach animus depravity, while exhibiting wide range of characteristic disorders, such as cerebration, perception, ego-consciousness, emotion and desire".

As a specific method for measuring the amount of EGF in serum, there may be mentioned, for example, a method comprising the steps of:
(1) immobilizing an anti-EGF antibody to a solid phase such as polystyrene, nylon, glass, silicone rubber or Sepharose;
(2) adding or contacting serum of a patient to be diagnosed to or with the solid phase;
(3) washing the solid phase;
(4) adding or contacting a labeled anti-EGF antibody to or with the solid phase; and
(5) measuring the amount of EGF using said labeling.

As a more specific method for measurement of EGF content in serum, there may be mentioned, for example, a method comprising the steps of:
(1) immobilizing an anti-EGF antibody to a solid phase such as polystyrene, nylon, glass, silicone rubber or Sepharose;
(2) adding or contacting the serum of a patient to be diagnosed to or with the solid phase;
(3) washing the solid phase;
(4) adding or contacting biotin modified anti-EGF antibody to or with the solid phase;
(5) adding or contacting labeled avidin to or with the solid phase; and
(6) measuring the amount of EGF using said labeling

As a further specific method for measuring the amount of EGF in serum, there may be mentioned, for example, a method comprising the steps of:
(1) immobilizing an anti-EGF antibody to a solid phase such as polystyrene, nylon, glass, silicone rubber or Sepharose;
(2) adding or contacting the serum of a patient to be diagnosed to or with the solid phase;
(3) washing the solid phase;
(4) adding or contacting 2,4-dinitrophenol modified anti-EGF antibody to or with the solid phase;
(5) adding or contacting labeled anti-2,4-dinitrophenol antibody to or with the solid phase; and
(6) measuring the amount of EGF using said labeling.

As the shape of the solid phase, a microsphere, a well, a test tube may be mentioned.

EGF, used as an antigen or standard for ELISA, is commercially available or can be produced by the following method.

When a technique of genetic engineering is used, a gene encoding EGF is inserted into a suitable vector, it is introduced into a suitable host to perform transformation. Then the objective recombinant EGF can be obtained from cells of the transformant or the culture medium (for example, Biotecnol. Appl. Biochem., 2000, Jun; 31 (Pt 3): 245-248). This method is suitable to achieve uniform and massive production of EGF. The above-mentioned host cell is not specifically limited, and various kinds of host cells conventionally used in the technique of genetic engineering, such as Escherichia coli, Bacillus subtilis, yeast, a plant cell or an animal cell, may be utilized.

The anti-EGF antibody can be prepared using EGF or its partial peptide as an antigen, by immunization of rabbit, chicken or turkey by the antigen. In the case of turkey, purified EGF (200 µg) is mixed with a complete Freund's adjuvant to be administered subcutaneously. Immunization is performed every one month and this operation is repeated until the titer reaches to a suitable value. Serum is obtained from the animal at this point.

The labeled anti-EGF antibody can be prepared by reacting an anti-EGF antibody with a biotinylating reagent (NHS-LC-Biotin, Pirece Co.) or using a commercially available kit of peroxidase attached with a cross-linking agent (Maleimide activated HRP, Pirce Co.).

Moreover, the assay kit of the present invention enables diagnosis of schizophrenia. That is, serum can be prepared from human blood, and the amount of EGF in serum can be determined by various methods. Then schizophrenia can be diagnosed by judging whether the determined value is included within the range of EGF value of normal control or not. As shown in Examples mentioned below, serum EGF content in schizophrenic patients significantly lowered as compared with the control group. Thus, the diagnostic method of schizophrenia is within the scope of the present invention, the method comprises the steps of collecting blood from human being and measuring the serum EGF content, then conducting diagnosis that the human being is schizophrenia in the case that the serum EGF content is 1/2 or less of the average value of the serum EGF content of the normal control group measured in the same manner, or conducting diagnosis that the human being is not schizophrenia in the case that the serum EGF content is twice or more of the average value of the serum EGF content of the schizophrenic group. From Examples as mentioned below, the serum EGF content of schizophrenic human being is preferably 200 pg/ml or less. More specifically, the diagnostic method of schizophrenia is within the scope of the present invention, the method comprises the steps of collecting blood from human being and measuring the serum EGF content using the diagnosis kit according to this invention which comprises a reaction vessel with fixed anti-epidermal EGF antibody and a labeled anti-EGF factor antibody, conducting diagnosis that the human being is schizophrenia in the case that the serum EGF content is 1/2 or less of the serum EGF content of the average value of normal control group measured in the same manner, or conducting diagnosis that the human being is not schizophrenia in the case that the serum EGF content is twice or more of the average value of the serum EGF content of the schizophrenic group.

By using the assay kit of the present invention, the serum EGF content can be measured not only in human being but also in schizophrenic model animal. A method for diagnosis of schizophrenia by measuring serum EGF content using the kit according to the present invention, not only in human being but also in an animal, is also within the scope of the present invention. Moreover, use of such a model animal enables development of a therapeutic medicament of schizophrenia and evaluation of the medical effect of a medicament. That is, the assay kit of the present invention is available as a powerful tool for development of a therapeutic medicament of schizophrenia or evaluation of medical effect of a medicament, since it realizes development and evaluation of medical effect of an anti-schizophrenic drug in vivo.

### EXAMPLES

In the following, the present invention is explained according to the Examples. Blood was collected with the consent of the patient himself/herself or family of the patient. The serum was optionally diluted with phosphate buffer containing protease inhibitor according to the conventional method. This sample was added to a 96-well plate coated with anti-EGF antibody (100 ng/well). EGF was added at the concentration of 1 to 30 pg/well and these were used as a standard for quantitative analysis. These were allowed to stand at room temperature overnight, then the sample was discarded and the wells were washed with the same buffer. Biotinylated anti-EGF antibody (30 ng/ml) was added and the mixture was allowed to stand at room temperature overnight. This secondary antibody was removed and the wells were washed, streptoavidin-galactosidase properly diluted by said buffer (approximately several 100-fold to several ten-thousands-fold in general) was added, and the mixture was allowed to stand at room temperature for several hours. This tertiary antibody was removed and the wells were washed, substrate for color development of galactosidase (200 µM 4-methyl umbellypheryl-(D-galactoside/50 mM sodium phosphate, pH 7.3, 10 mM MgCl₂) was added. The color was developed until a suitable standard curve can be prepared. In the case of a fluorescent substrate like a color developing substrate of this kind, fluorescent intensity at 448 nm was measured under excitation light at 364 nm, using a fluorescent plate reader. A plural number of wells were used per one sample and the concentration of EGF in the sample was calculated from the calibration curve.

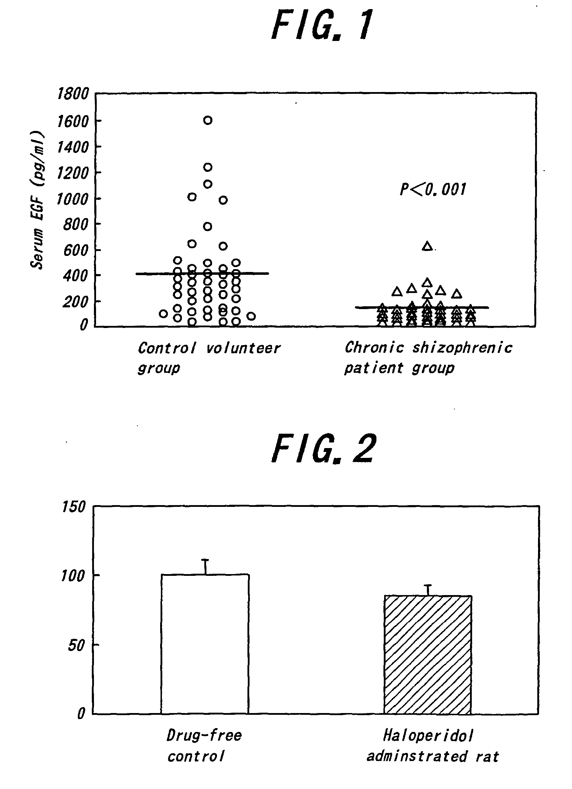

In the chronic schizophrenic group (45 individuals) and the control volunteer group (45 individuals) with sex- and average age-matched, the EGF level in human fresh serum was measured by the two site ELISA method. In the chronic schizophrenic group, The average of EGF level was 136 pg/ml and S.D. was 111. Meanwhile, in the control volunteer group, the average was 392 pg/ml and S.D was 343. When the serum EGF levels were compared between these two groups, it was revealed that the level decreased significantly (p<0.001) in the schizophrenia group (Fig. 1). In Fig. 1, the serum EGF level of the control group was shown in the left and the serum EGF level of the schizophrenic group was shown in the right, respectively. To investigate the effect of medicament on the serum EGF level, rats with 2 weeks administration of haloperidol (0.5 mg/kg) were prepared. Then the bloods from these rats and control rats were collected, and EGF levels in the sera were measured by two site ELISA method, in the same manner as in human serum. When compared between the two groups, no significant difference was recognized (p>0.05) (Fig. 2). In Fig. 2, the serum EGF level of the control group was shown in the left and that of the haloperidol administered group was shown in the right, respectively. From the results shown in Fig. 2, it is assumed that decreased EGF level observed in the chronic schizophrenic group is not due to the effect of chronic administration of medicament.

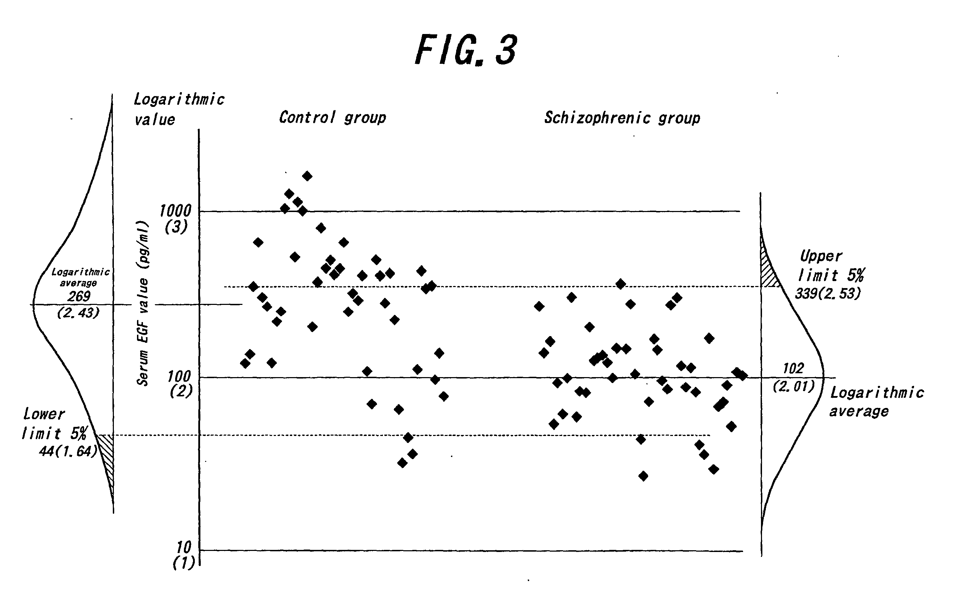

Statistic test: The EGF values of these three groups do not exhibit normal distribution, thus it is impossible to test the abnormality of the individual EGF value as such. The logarithms (log values) of these EGF values exhibit normal distribution as shown in the table. Fig. 3 is a drawing showing the data by a diagram. The average of logarithmic EGF values of the control group is 2.43 and the standard deviation is 0.41. The abnormal value estimated from this distribution, i.e., the level lower than 5% lower limit is 2.43-(0.41x1.9)=1.64 (44 pg/ml). Therefore, 5 individuals among 45 individuals of the chronic schizophrenic patents are judged to be "out of the normal value range". Moreover, the average of logarithmic EGF values of the schizophrenic group is 2.01 and the standard deviation is 0.275. The upper abnormal value estimated from this distribution, i.e., the level upper than 5% upper limit is 2.01+(0.275x1.9)=2.53 (339 pg/ml). Therefore, 21 individuals among 45 individuals of the control are judged to be "not in the range of the abnormal value". Incidentally, the remaining individuals are pseudo-positive and can not be judged.

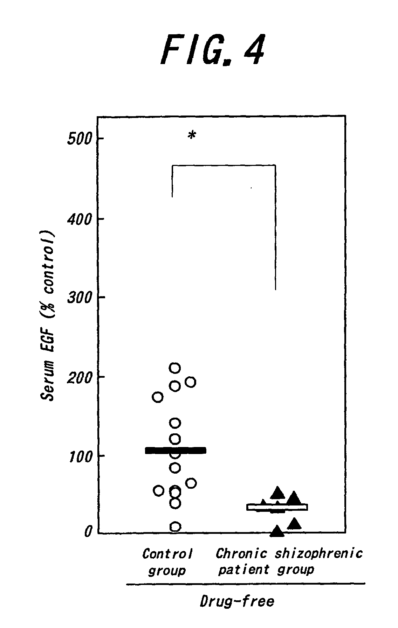

In Fig. 1, the result was obtained on the patients with medication. Therefore, to investigate the effect of medication on the EGF level, serum EGF level was measured on the control volunteer group and on the drug-native chronic schizophrenic patients (drug-free patient group). The EGF level was measured on fourteen individuals of control volunteer group (open circles) and drug-free patient group (solid triangles) by the method as previously described, and the results are shown in Fig. 4. The vertical axis in Fig. 4 indicates serum EGF level of the each individual of the control volunteer group and the drug-free patient group, when the average serum EGF level in the control volunteer group was calibrated to 100. The serum EGF level was compared between these two groups, indicating that the serum EGF level decreased significantly (p<0.001) in the drug-free chronic schizophrenic group, and the result was same as that of Fig. 1. Therefore, it was confirmed that the decreased serum EGF level in the chronic schizophrenic patient group was not due to the effect of medication.

According to the present invention, a diagnostic kit for schizophrenia is provided and the diagnostic kit comprises measurement of serum EGF level using anti-EGF antibody.

## Claims

1. A diagnostic kit for schizophrenia, comprising a solid phase, an anti-epidermal growth factor (EGF) antibody immobilized to said solid phase and a labeled anti-EGF antibody.

2. The diagnostic kit according to Claim 1, wherein said labeling is enzyme labeling, fluorescent labeling or radioisotope labeling.

3. The diagnostic kit according to Claim 1, wherein enzyme for said enzyme labeling is selected from the group consisting of peroxidase, β-D-galactosidase, alkaline phosphatase and glucose-6-phosphate dehydrogenase.

4. A diagnostic kit for schizophrenia comprising a solid phase, an anti-EGF andibody immobilized to said solid phase, a biotin-modified anti-EGF antibody and labeled avidine which reacts with said biotin.

5. A diagnostic kit for schizophrenia comprising a solid phase, an anti-EGF antibody immobilized to the solid phase, a 2,4-dinitrophenol modified anti-EGF antibody and a labeled anti-2,4-dinitrophenol antibody which reacts with said 2,4-dinitrophenol.

6. The diagnostic kit for schizophrenia according to any one of Claims 1 to 5, wherein serum EGF concentration is measured using said anti-EGF antibody.

7. The diagnostic kit for schizophrenia according to any one of Claims 1 to 6, wherein said schizophrenia is acute schizophrenia or chronic schizophrenia.

8. A method for diagnosis of schizophrenia in a test subject, the method comprising the steps of:
(1) collecting blood from said test subject and normal control group, then obtaining serum of each individual from blood of each individual;
(2) measuring EGF concentration in serum of each individual using an anti-EGF antibody; and
(3) condicting diagnosis that said test subject is schizophrenia in the case that the concentration of EGF in said serum of said test subject is 1/2 or less compared with the average concentration of EGF in said normal control group.

9. A method for diagnosis of schizophrenia in a test subject, the method comprising the steps of:
(1) collecting blood from said test subject and schizophrenic group, then obtaining serum of each individual from said blood of each individual;
(2) measuring the concentration of EGF in said serum of each individual using an anti-EGF antibody; and
(3) conducting diagnosis that said test subject is not schizophrenia in the case that the concentration of EGF in said serum of said test subject is twice or more compared with the average concentration of EGF in said schizophrenic group.

10. The method for diagnosis of schizophrenia according to Claim 8 or Claim 9, comprising measurement of concentration of EGF in said serum using the diagnostic kit according to Claim 1.

11. The method for diagnosis of schizophrenia according to Claim 8 or Claim 9, wherein said schizophrenia is acute schizophrenia or chronic schizophrenia.
